# EUROPÄISCHE PATENTSCHRIFT

(11) **EP 1 185 520 B1**
(45) Veröffentlichungstag und Bekanntmachung des Hinweises auf die Patenterteilung: **17.03.2004**
(21) Anmeldenummer: 00925194.3
(22) Anmeldetag: 11.04.2000
(51) Int. Cl.: C07D 277/04

(54) **VERFAHREN ZUR HERSTELLUNG VON THIAZOLIDIN**
METHOD FOR THE PRODUCTION OF THIAZOLIDIN
PROCEDE DE PREPARATION DE THIAZOLIDINE

(30) Priorität: 10.06.1999 DE 19926233
(43) Veröffentlichungstag der Anmeldung: 13.03.2002
(73) Patentinhaber: Probiodrug AG, 06120 Halle/Saale (DE)
(72) Erfinder: DEMUTH, Hans-Ulrich, 06114 Halle/Saale (DE); KRUBER, Susanne, 06114 Halle (DE)
(74) Vertreter: Forstmeyer, Dietmar, Dr. rer. nat., Dipl.-Chem.
(86) Internationale Anmeldenummer: PCT/EP2000/003213
(87) Internationale Veröffentlichungsnummer: WO 2000/076986

(56) Entgegenhaltungen:
- EP-A- 0 054 409
- EP-A- 0 695 744
- CH-A- 590 857
- US-A- 3 594 377

## Beschreibung

Die Erfindung betrifft die einfache und technisch leicht durchführbare Herstellung von Thiazolidin-Base und seinen Salzen.

Thiazolidin kann als Zwischenprodukt zur Synthese von Aminoacyl- und Peptidyl-Thiazolididen dienen, die als Enzyminhibitoren sowohl diagnostischen als auch therapeutischen Wert besitzen [DEMUTH, H.-U., J. Enzyme Inhibition 3, 249 (1990)].

Da sich Aminoacyl Thiazolidide u.a. zur Regulation des Blutglukosespiegels bei Säugern eignen, ist die Darstellung dieser Verbindungen einschließlich ihrer Ausgangsstoffe in kostensparenden, technisch anwendbaren Verfahren von medizinischem, pharmazeutischem und wirtschaftlichem Interesse [vgl. DE 19 616 486].

Es ist bekannt, daß man Thiazolidin und Thiazolidinderivate gewinnen kann, indem man Aldehyde mit Aminoethylsulphat oder - halogeniden mit Natriumsulfid in wäßriger Lösung unter exzessiver Energiezufuhr mehrere Stunden unter Rückfluß erhitzt. Die Ausbeuten betragen ca. 60 % der Theorie [vgl. US 4 584 407].

Demgegenüber war es die Aufgabe der vorliegenden Erfindung, ein Verfahren zur Herstellung von Thiazolidin-Base bzw. deren Salzen bereitzustellen, bei dem keine exzessive Energiezufuhr nötig ist.

Erfindungsgemäß wird nunmehr ein Verfahren zur Herstellung von Thiazolidin-Base und deren Salzen bereitgestellt, das dadurch gekennzeichnet ist, daß Hexamethylentetramin der Formel (I) 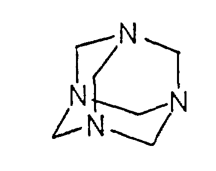 mit Cysteamin oder seinen Salzen der Formel (II) 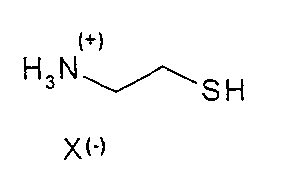 worin X⁽⁻⁾ einen Säurerest darstellt, umgesetzt wird, wobei bevorzugt wird, wenn X⁽⁻⁾ ein Halogenid oder Sulfat ist.

Es ist als ausgesprochen überraschend zu werten, daß man nach diesem Verfahren die freie Base Thiazolidin und deren Salze in sehr hoher Reinheit und Ausbeute erhält, ohne daß bei der Reaktion größere Wärmemengen zugeführt werden müssen. Dies stellt einen wirtschaftlichen und technologischen Vorzug des erfindungsgemäßen Verfahrens insbesondere bei der technischen Herstellung von Thiazolidin dar [vgl. EP 0054409].

Erfindungsgemäß kann die Umsetzung z.B. in einem polaren Lösungsmittel wie einem Alkohol erfolgen. Bevorzugte Lösungsmittel sind Methanol und/oder Ethanol.

Als ein weiterer wirtschaftlicher und technologischer Vorzug des erfindungsgemäßen Verfahrens bei der technischen Herstellung von Thiazolidin ist die Tatsache zu werten, daß Hexamethylentetramin hinsichtlich des pharmazeutischen Einsatzes der Folgeprodukte des Thiazolidins unbedenklich ist, da es pharmazeutisch unbedenklich ist: lange Zeit wurde es als Harndesinfiziens und zur Lebensmittelkonservierung verwendet [vgl. Mutschler, Arzneimittelwirkungen, S.572f., Stuttgart: Wissenschaftliche Verlagsges. (1986)].

Vorzugsweise wird bei der Umsetzung Ammoniak vorgelegt und/oder zugesetzt. Dadurch kann die Synthese bis zur Stufe der freien Base in einem Schritt erfolgen [vgl. Ratner, S., Clarke, H.T., J. Am. Chem. Soc. 59, S.200-206 (1937)], so daß zusätzliche umständliche und teure Reaktionsschritte vermieden werden können.

Das erfindungsgemäße Verfahren, welches sowohl für den Labormaßstab als auch den großtechnischen Einsatz entwickelt wurde, wird z.B. so durchgeführt, daß man zu einer vorzugsweise methanolischen Lösung eines Cysteaminsalzes auf einmal oder in mehreren Portionen Hexamethylentetramin als Feststoff oder in einem Lösungsmittel gelöst gibt. Das Gemisch kann mehrere Stunden bei Raumtemperatur, oder aber auch bei Temperaturen um 30-35 °C gerührt werden. Die angegebene Dosierung kann auch in umgekehrter Reihenfolge erfolgen.

Das erfindungsgemäße Verfahren muß nicht unbedingt wie andere Verfahren unter Inertgas durchgeführt werden [vgl. EP 0695744].

Das nach dem erfindungsgemäßen Verfahren hergestellte Thiazolidin kann als Ausgangsstoff zur Herstellung von pharmazeutisch anwendbaren Wirkstoffen verwendet werden. Die Erfindung wird anhand des folgenden Beispiels verdeutlicht.

### Beispiel

Zu einer Lösung von 1,358 kg (12 mol) Cysteaminhydrochlorid, vorgelegt in 1,8 l Methanol bei 30 - 35 °C, werden bei einer Reaktionstemperatur von 30 - 35 °C 291,59 g (2,08 mol) Urotropin in zwei Portionen gegeben. Nach der Zugabe der ersten Portion des Hexamethylentetramins kann eine deutliche Exothermie (∼45 °C) und Violettfärbung beobachtet werden, die Reaktionsmischung wird gekühlt. Ammoniumchlorid beginnt grob auszufallen. Nach Abklingen der exothermen Reaktion (1,5 h) wird die zweite Portion Hexamethylentetramin addiert. In den Ansatz wird Ammoniak bis zur Sättigung eingeleitet, 700 ml tert-Butyl-methylether werden addiert.

Das quantitative Ausfallen von Ammoniumchlorid kann als Improzeßkontrolle gewertet werden. NH₄Cl wird abgesaugt und der Filterkuchen mit der Reaktionslösung nachgewaschen. 300 ml Aminoethylethanolamin werden als Sumpfbildner in die Lösung gegeben. Thiazolidin wird destillativ gereinigt, Sdp.: 60-70 °C, 8-10 mbar. Die hochreine Substanz kann mit einer Ausbeute von 88-93 % erhalten werden.
^{**1**}**H-NMR** (200 MHz, D₂O) δ (ppm) = 2,80-2,83 (t, ³J=6,45 Hz, 2H, NCH₂CH₂), 3,04-3,19 (t, ³J= 6,45 Hz, 2H, CH₂CH₂S), 4,05 (s, 2H, NCH₂S
^{**13**}**C-NMR** (100,5 MHz, DMSO-d₆) δ (ppm) 30,69 (s, NCH₂CH₂) 47,31 (s, CH₂CH₂S), 47.95 (s, NCH₂S)
**MS** (MALDI-TOF) 89 (M+H)
**EA:** C₃H₇NS ber.: C = 40,44 % gef.: C = 40,27 %
H = 7,91 % H = 8,02 %
N = 15,72 % N = 15, 90 %
S = 35,91 % S = 35,73 %

## Patentansprüche

1. Verfahren zur Herstellung von Thiazolidin-Base und deren Salzen **dadurch gekennzeichnet, daß** Hexamethylentetramin der Formel (I) 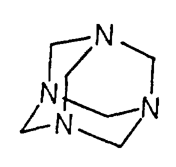 mit Cysteamin oder seinen Salzen der Formel (II) 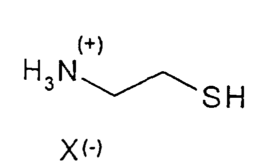 worin X⁽⁻⁾ einen Säurerest darstellt, umgesetzt wird.

2. Verfahren nach Anspruch 1, **dadurch gekennzeichnet, daß** X(-) ein Halogenid oder Sulfat ist.

3. Verfahren nach einem der vorstehenden Ansprüche, **dadurch gekennzeichnet, daß** die Umsetzung in einem polaren Lösungsmittel erfolgt.

4. Verfahren nach Anspruch 3, **dadurch gekennzeichnet, daß** das Lösungsmittel ein Alkohol ist.

5. Verfahren nach Anspruch 3 oder 4, **dadurch gekennzeichnet, daß** das Lösungsmittel Methanol oder Ethanol ist.

6. Verfahren nach Anspruch 3 oder 4, **dadurch gekennzeichnet, daß** vor und/oder während der Umsetzung Ammoniak zugesetzt wird.

7. Verfahren nach einem der vorstehenden Ansprüche, **dadurch gekennzeichnet, daß** Ammoniumsalz abgetrennt und/oder das Produkt destilliert wird.

## Claims

1. A process for the production of thiazolidine base and salts thereof, wherein hexamethylenetetramine of formula (I) 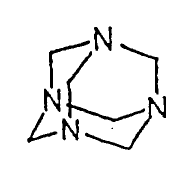 is caused to react with cysteamine or salts thereof of formula (II) 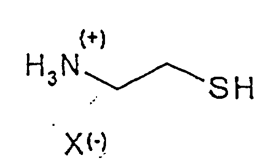 in which X⁽⁻⁾ represents an acid residue.

2. A process as defined in claim 1, wherein X⁽⁻⁾ is a halide or sulfate.

3. A process as defined in any of the previous claims, wherein the reaction is carried out in a polar solvent.

4. A process as defined in claim 3, wherein the solvent is an alcohol.

5. A process as defined in claim 3 or claim 4, wherein the solvent is methanol or ethanol.

6. A process as defined in claim 3 or claim 4, wherein ammonia is added before and/or during the reaction.

7. A process as defined in any of the previous claims, wherein ammonium salt is separated and/or the product is distilled.

## Revendications

1. Procédé de fabrication de base thiazolidine et de ses sels, **caractérisé en ce que** l'hexaméthylènetétramine de formule (I) 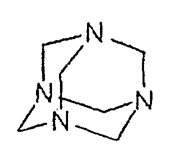 est transformée avec la cystéamine ou ses sels de formule (II) 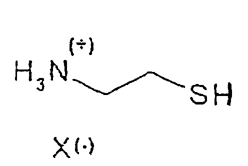 où X⁽⁻⁾ représente un résidu d'acide.

2. Procédé selon la revendication 1, **caractérisé en ce que** X⁽⁻⁾ est un halogénure ou un sulfate.

3. Procédé selon l'une des revendications précédentes, **caractérisé en ce que** la transformation a lieu dans un solvant polaire.

4. Procédé selon la revendication 3, **caractérisé en ce que** le solvant est un alcool.

5. Procédé selon les revendications 3 ou 4, **caractérisé en ce que** le solvant est le méthanol ou l'éthanol.

6. Procédé selon les revendications 3 ou 4, **caractérisé en ce que** avant et/ou pendant la transformation, on ajoute de l'ammoniac.

7. Procédé selon l'une des revendications précédentes, **caractérisé en ce que** le sel d'ammonium est séparé et/ou le produit est distillé.
